# EUROPEAN PATENT SPECIFICATION

(11) **EP 3 852 878 B1**
(45) Date of publication and mention of the grant of the patent: **23.08.2023**
(21) Application number: 19856432.0
(22) Date of filing: 19.09.2019
(51) Int. Cl.: A61P 33/00, C07C 309/31, C07D 239/50, A61K 31/505

(54) **DICYCLANIL SALT WITH DODECYLBENZENESULFONATE AND USE OF SUCH A SALT IN COMBATING PARASITES**
DICYCLANIL-SALZ MIT DODECYLBENZOLSULFONAT UND VERWENDUNG EINES SOLCHEN SALZES ZUR BEKÄMPFUNG VON PARASITEN
NOUVEAU SEL DE DICYCLANIL AVEC SULFONATE DE DODECYLBENZENE, PROCÉDÉ DE PRÉPARATION D'UN TEL SEL, COMPOSITIONS CONTENANT UN TEL SEL, ET UTILISATION D'UN TEL SEL POUR COMBATTRE DES PARASITES

(30) Priority: 20.09.2018 PL 42714318
(43) Date of publication of application: 28.07.2021
(73) Proprietor: ICB Pharma Spólka Jawna, 43-602 Jaworzno (PL)
(72) Inventor: SWIETOSLAWSKI, Janusz, 43-600 Jaworzno (PL); WIECZOREK, Wojciech, 43-502 Czechowice-Dziedzice (PL)
(74) Representative: Lukaszyk, Szymon
(86) International application number: PCT/PL2019/050052
(87) International publication number: WO 2020/060429

(56) References cited:
- AU-B1- 2007 202 548
- DE-A1- 2 417 008
- US-A- 4 783 468

## Description

The present invention relates to 6-diamino-2-cyclopropylaminopyrimidine-5-carbonitrile (dicyclanil) dodecylbenzenesulfonate, a method of preparing dicyclanil dodecylbenzenesulfonate, antiparasitic, preferably antilarvicidal compositions comprising dicyclanil dodecylbenzenesulfonate, and a use of dicyclanil dodecylbenzenesulfonate in combating and controlling of parasites, in particular diptera larvae, in particular larvae of blow flies of the *Calliphoridae* family, such as *Lucilia cuprina* and *Lucilia sericata*, in humans and animals.

### BACKGROUND OF THE INVENTION

Dicyclanil (IUPAC name: 4,6-diamino-2-cyolopropylaminopyrimidine-5-carbonitrile, CAS number; 112636 83-6) and its physiologically acceptable salts have been known since the 1980s as ectoparasite control agents, in particular as larvicides against diptera larvae, in particular larvae of blow flies, but also as larvicides against larvae of house flies, mosquitos and fleas.

Dicyclanil is very slightly soluble in water (solubility is approximately 0.3% by weight at 20 °C) and such a poor water solubility is an advantage thereof as an anti-ectoparasite agent, as it is not rained washed out quickly by water (rain) from a surface onto which it was applied. However, due to its rapid absorption into the animal's body and rapid metabolization, the efficiency of dicyclanil as such alone as an antiparasitic agent for combating ectoparasites has been shown to be relatively low in practice, in particular unexpectedly low compared to the efficiency achieved by dicyclanil in *in vitro* tests.

Therefore efforts have been made in order to produce dicyclanil derivatives featuring an improved performance under practical conditions.

For example patent publication US 4,783,468 discloses dicyclanil salts of methanesulfonic acid and benzenesulfonic acid which are useful being dissolved in a solvent as spray-on preparations in a treatment of sheep myiasis.

Patent publication EP1007516B1, in turn, discloses a crystalline modification of D dicyclanil. A commercially available product marketed as "CLICK Pour-On" by Novartís Co., is a ready-to-use aqueous suspoemulsion (SE) of a polymorphic D form of dicyclanil which is a spray-on/pour-on formulation (50 g/l).

international patent publication WO 2009/118312 A1 discloses dicyclanil polyethylene glycol (PEG) solutions. This publication, however, does not disclose data on a stability of such formulations at low temperatures (of about 0 °C), as well as on a resistance of such formulations against rinsing out with water from skin or hair of animals treated with the solution of this invention. Taking into account that PEG glycols are of a significantly limited volatility and highly soluble in water, it should be presumed that the disclosed preparation is easily washable by rain, and therefore its use is considerably limited.

Patent publication AU 200720254881 discloses dicyclanil solutions in particular selected solvents and some carboxylic acids. The commercially available product marketed as "StrikeForce-S" by Jurox Co., constituting a preparation according to this patent and containing a solution of 50 g/l of dicyclanil, 10-20% of lactic acid and 1-10% of surfactant (ethoxylated octylphenol), is recommended for protecting sheep against myiasis caused by a blow fly species (*Lilla cuprina*) using a spraying application, providing a protection for a period of 18 to 24 weeks.

Patent publication AU 201029533882 discloses aqueous suspensions of dicyclanil with lignosulfonates used to prevent a recrystallization of dicyclanil and a resulting sedimentation and/or separation of such suspensions. As disclosed in the test conducted for 4 weeks at room temperature, the optimum pH amounts from 3.99 to 5.86 (no data for the CIPAC test at 54°C/14 days).

All of the prior art dicyclanil compounds as used in practice are relatively well soluble in water and therefore their period of effectiveness when applied to animals kept in open-range and thus exposed to an influence of rain is relatively short. The period of effectiveness of the known dicyclanil formulations is, in particular, too short relative to the period of an effective action required to prevent spreading of sheep myiasis.

In addition, a problem of dicyclanil crystal growth and a stability problem resulting therefrom appears for many formulations containing dicyclanil in a form of an aqueous dispersion, and in particular a problem with regard to sedimentation and delamination of such formulations during a storage thereof.

The object of the invention has been to provide a form of dicyclanil effective in controlling parasites, which would be sparingly soluble in water and obtainable in a simple manner, preferably *in situ,* directly in compositions for combating and controlling parasites, in particular diptera larvae, especially blow fly larvae, in humans or animals.

### SUMMARY OF THE INVENTION

It has been unexpectedly discovered, that dicyclanil with dodecylbenzenesulfonic acid (iso- or n- or as a mixture of isomers) forms a very slightly water-soluble salt of dodecylbenzenesulfonic acid and 4,6-diamino-2-cylopropylaminopyrimidine-5-carbonitrile (dicyclanil dodecylbenzenesulfonate), which is biologically effective in controlling parasites, and is especially effective as larvicide against diptera larvae, in particular against larvae of blow flies of the *Calliphoridae* family, especially against larvae of *Lucilia cuprina* and *Lucilia sericata.*

Dicyclanil dodecylbenzenesulfonate is particularly effective in controlling sheep myiasis caused by *Lucilia cuprina* and *Lucilia sericata.*

The present invention provides therefore 4,6-diamino-2-cyclopropylaminopyrimidine-5-carbonitrile dodecylbenzenesulfonate (also referred to herein in abbreviate dicyclanil dodecylbenzenesulfonate) which is a new salt of 4,6-diamino-2-cyclopropyiaminopyrimidine-5-carbonitrile and dodecylbenzenesulfonic acid.

in contrast to the prior art dicyclanil compounds (salts), such as for example dicyclanil salts with methanesulfonic acid or benzenesulfonic acid disclosed in patent publication US4783468, which are readily soluble in water and therefore easily washed out by rain from sheep's skin and fur, the dicyclanil salt according to the invention is very poorly soluble in water, so that it remains on a skin and fur of an animal for a long time,

The present dicyclanil dodecylbenzenesulfonate may be preferably obtained by the reaction of 4,6-diamino-2-cyclopropylaminopyrimidine-5-carbonitrile with dodecylbenzenesulfonic acid.

The reaction is preferably carried out in a mixture of dodecylbenzenesulfonic acid with dicyclanil having an acid/dicyclanil molar ratio ranging from 0.50 to 1.10,

Setting up a certain molar excess of dodecylbenzenesulfonic acid in such this mixture ensures, on the one hand, the maximum use of dicyclanil. On the other hand, in the case where a method of preparing dicyclanil dodecylbenzenesulfonate according to the invention takes place *in situ* in a ready-to-use composition, the unreacted dodecylbenzenesulfonic acid is an undesirable toxic substance that may be irritating to an animal with respect to which such a composition will be used and which may reduce the composition stability during storage.

Therefore, in a case of a preparation of the present dicyclanil dodecylbenzenesulfonate salt directly in a final composition, it is advantageous to use a molar deficiency of dodecylbenzenesulfonic acid (an excess of dicyclanil). In particular, it is preferred to use a mixture of dodecylbenzenesulfonic acid with dicyclanil having an acid/dicyclanil molar ratio amounting from 0.70 to 0.98.

The inventors of the present invention have surprisingly discovered that said dicyclanil dodecylbenzenesulfonate facilitates a dissolution of dicyclanil in a glycol ether/water solvent system. Therefore, preparing the salt according to the present invention using a mixture of dodecylbenzenesulfonic acid and dicyclanil in a molar excess is particularly preferably carried out in a glycol ether/water solvent system, since an dicyclanil excess will be then well dissolved and crystallization will not cause problems with a stability of such a composition.

Furthermore, it has been surprisingly discovered that a solubility of the salt of the present invention in water (an elution) can be advantageously further reduced by adding castor oil to the compositions comprising the present dicyclanil dodecylbenzenesulfonate salt.

Additionally castor oil advantageously improves a stability of such a composition during storage at low temperatures and because of its lipophilicity improves a penetration of the composition into a skin and spreading it on a skin.

Exceptionally preferred glycol ethers for use according to the present invention are dipropylene glycol monomethyl ether (such as, for example, the commercially available product "Dowanol DPM") and diethylene glycol monoethyl ether ("Carbitol Solvent).

The present invention furthermore provides an antiparasitic composition, preferably a larvicide composition, comprising the present salt of dodecylbenzenesulfonic acid and 4,6-diamino-2-cyclopropylaminopyrimidine-5-carbonitrile.

The content of 4,6-diamino-2-cyclopropyiaminopyrimidine-5-carbonitrile dodecylbenzenesulfonate in such a composition according to the invention amounts preferably from 1 to 25 wt. %, and more preferably from 10 to 15 wt.%. of the composition.

The composition according to the present invention preferably comprises castor oil in an amount of from 1 to 10% by weight of the composition.

In addition, the composition preferably further comprises a glycol ether selected from the group comprising dipropylene glycol monomethyl ether and diethylene glycol monoethyl ether and/or mixtures thereof in any ratio, in an amount of from 50 to 90% by weight of the composition and water in an amount of up to 10% by weight of the composition.

The composition of the invention is preferably in a form of a water-soluble concentrate (a so--called SL formulation).

The present invention furthermore provides 4,6-diamino-2-cyolopropylaminopyrimidine-5-carbonitrile dodecylbenzenesulfonate for use in combating and controlling of parasites in humans and animals, in particular diptera larvae, in particular larvae of blow flies of the *Calliphoridae* family, in particular larvae of *Lucilia cuprina* and *Lucilia sericata.* An employment of a local application of a composition of the present invention on a human or animal body is preferred.

### DETAILED DESCRIPTION OF THE INVENTION

The information provided above will obviously enable a skilled technician employing the present invention in the broadest scope of the patent claims. Therefore, the following embodiments of the present invention are set forth for purposes of illustration only, for a better understanding of the invention. Therefore, the embodiments do not limit the scope of protection of the present invention as defined in the claims.

### Physicochemical tests

### Synthesis of dicyclanil dodecylbenzenesulfonate

For the purpose of physicochemical tests, the synthesis of dicyclanil dodecylbenzenesulfonate was carried out according to the following reaction scheme; 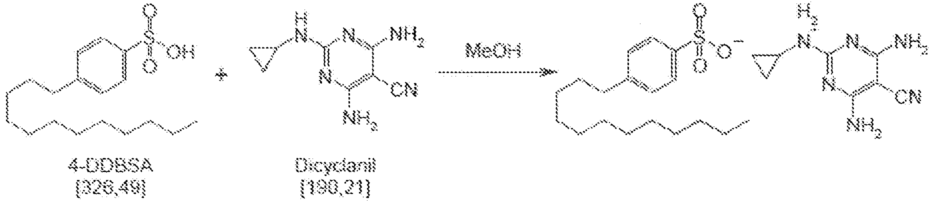 as described below.

In an Erlenmeyer flask, 1000 ml, a suspension of 50.04 g (ca. 0.263 mol) of dicyclanil in 370 ml of methanol was magnetically stirred (IKA RH basic; dipole 8 × 40). 85.73 g of 4-dodecylbenzenesulfonic acid (Aldrich 44198, 96.4%, 0.253 mol) was slowly added over about 40 minutes. Stirring was continued until obtaining a complete dissolution of the precipitate yielding a slightly opalescent, pale yellowbrown solution.

The agitator speed was increased and very slowly, during about 135 min, 434 g of demineralized water was added. After the addition of water was complete, the mixture was stirred for a further 35 min and then cooled to 15 °C.

The resulting, off-white, suspension was filtered; then washed with 200 ml of cooled 30% aqueous methanol and 500 ml of demineralized water. The wet solid precipitate was dried in a vacuum desiccator at 35-40 °C for 9 hours to give 133.1 g of dicyclanil dodecylbenzenesulfonate as a white powder, mp 173.0-174.7°C.

### Spectroscopic spectrum

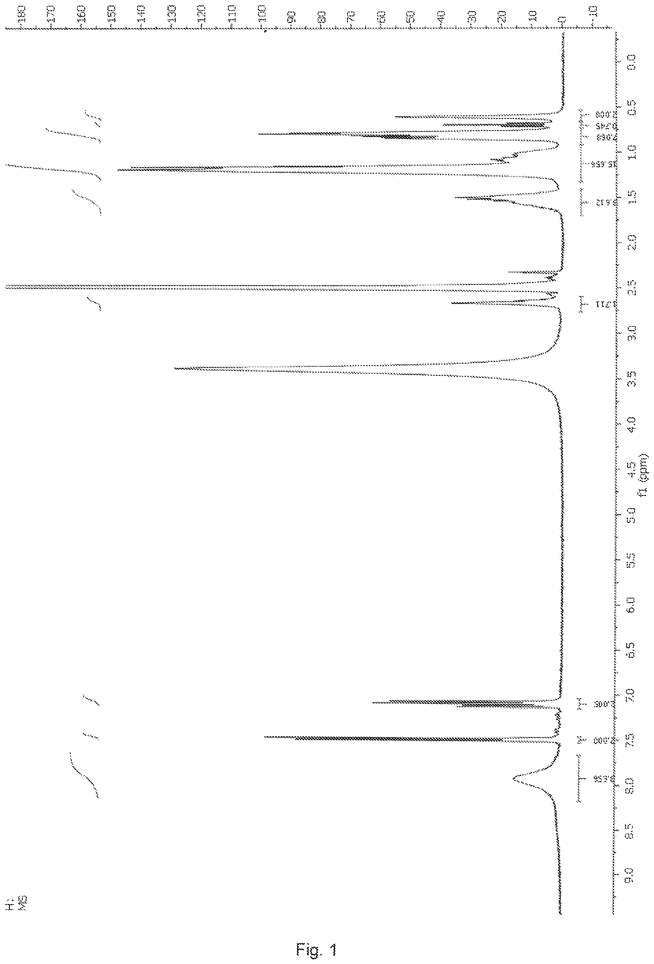

A spectroscopic spectrum of the dicyclanil dodecylbenzenesulfonate synthesized as described above was determined using ¹H NMR nuclear magnetic resonance spectroscopy using dimethylsulfoxide-D₆ as a solvent. The ¹H NMR spectrum in DMSO-D₆ obtained for the dicyclanil dodecylbenzenesulfonate is shown in Fig. 1.

### Solubility tests

To document the unexpectedly low water solubility of the present salt, i.e. dicyclanil dodecylbenzenesulfonate, comparative solubility tests were carried out after 4 and 24 hours of mixing of 10% aqueous dispersion of the present salt, as well as free dicyclanil alone, in a laboratory mixer at 20 °C, after which the sample was filtered and the free/bound dicyclanil was determined in a filtrate by a HPLC method.

The test results are summarized in the following Table 1.

**Table 1, Comparative solubility tests**

| Substance | Solubility in water after 4 h of mixing [%] | Solubility in water after 24 h of mixing [%] |
|---|---|---|
| Technical dicyclanil TC 99.0% | 0.312 | 0.31 7 |
| Dicyclanil dodecylbenzenesulfonate according to the present invention | 0.0490 | 0.0495 |

The results obtained confirm that the present salt dissolves in water not only much less than known dicyclanil salts (such as for example a salt with methanesulfonic acid with a solubility of > 5%), but also much less than the free dicyclanil alone, which until now was considered very slightly soluble in water. Due to such a poor solubility, the action of the dicyclanil dodecylbenzenesulfonate oil a skin and fur of animals will be substantially much longer than the prior art dicyclanil compounds used against parasites.

### Example 1

The following is an exemplary method of preparing the dicyclanil dodecylbenzenesulfonate under *in situ* conditions, directly yielding the P1 composition of the invention for controlling parasites. The method uses the ingredients listed in the following Table 2.

**Table 2. Ingredients for the preparation of the composition P1 and of the final composition P1**

| Ingredient | % by weight of the mixture of ingredients (according to the order of an addition) | % by weight of the composition P1 |
|---|---|---|
| Water | 6.30 | 6.30 |
| Cochineal red | 0.02 | 0.02 |
| Dowanol DPM (dipropylene glycol monomethyl ether) | 76.00 | 76.00 |
| BHT (antioxidant) | 0,08 | 0,08 |
| Dicyclanil (99%) | 5.10 | 1,10 |
| Dodecylbenzenesulfonic acid 98.5 % ^{∗} | 7.50 | 0.00 |
| Dodecylbenzenesulfonate of 4,6-diamino-2-cyclopropylaminopyrimidine-5-carbonitrile | - | 11.50 |
| Castor oil | 5.00 | 5.00 |

| | | |
|---|---|---|
| * molar ratio of dodecylbenzenesulfonic acid/dicyclanil = 0.85. | | |

Dicyclanil powder was poured into a solution of water, cochineal red, Dowanol and BHT with stirring with a high-cutting agitator. While stirring, dodecylbenzenesulfonic acid was added and stirring was continued for a further 5 minutes until the dicyclanil dissolved completely, while heating to 45 °C. Castor oil was then poured, and the solution was stirred again and cooled to room temperature.

As a result of this process, the composition P1 according to the present invention was obtained with a dicyclanil content (free + as a salt) of 50.4 g/l (12.60 wt %) of the composition, wherein the composition comprised 42.8 g/l (11.50 wt%) of dicyclanil in the form of the present salt of the invention with dodecylbenzenesulfonic acid.

The obtained composition P1 has the form of a clear red liquid of pH 2.88, a density of 0.991 kg/dm³ (at 20°C), and it is stable during storage at 0°C. Storage stability tests at 54 °C for 14 days showed that the initial dicyclanil content (50.4 g/l) in the P1 composition according to the invention decreased only by 2.18% to 49.3 g/l.

### Example 2

Table 3 below summarizes the exemplary compositions P2-P5 of the present invention for controlling ectoparasites, that contain the present dicyclanil dodecylbenzenesulfonate, as well as comparative compositions A1 (with free dicyclanil alone) and A2 (with the dicyclanil/lactic acid mixture as disclosed in AU 2007202548 B1); and the stability assessments obtained for these compositions expressed by describing the appearance of the composition at 20 °C and after holding for 48 hours at 0°C.

**Table 3. Ingredients of the exemplary compositions P2-P5 according to the invention and comparative compositions A1-A2**

| Ingredient | % by weight of the composition | | | | | |
|---|---|---|---|---|---|---|
| | P2 | P3 | P4 | P5 | A1 | A2 |
| Dicyclanil (99%) | 3.0 | 1.4 | 0.8 | 0.0 | 5 | 5 |
| Dodecylbenzenesulfonic acid | 0.0 | 0.0 | 0.0 | 0.6 | - | - - |
| Dicyclanil dodecylbenzenesulfonate | 7.0 | 9.6 | 11.2 | 13.4 | - | |
| Dowanol DPM | 80 | 80 | 80 | 80 | 80 | 80 |
| Water | 10 | 9 | 8 | 6 | 15 | 11.9 |
| Lactic acid (80%) | - | - | - | - | - | 3.1 |
| Sample appearance (20°C) | clear liquid | | | | sediment | |
| Sample appearance (0°C/48 h) | clear liquid | | | | sediment | |

All P2-P5 compositions according to the invention remained stable as a clear liquid, whereas in the prior art A1-A2 compositions a precipitate was found confirming an insufficient stability of the prior art preparations not allowing their practical use.

All of the P2-P5 compositions of the invention and the A1-A2 compositions were obtained in a manner analogous to the method described above for obtaining the P1 composition using the ingredients listed in the following Table 4.

**Table 4. Ingredients for preparing compositions P2-P5and A1-A2**

| Ingredient | % by weight of the mixture of ingredients | | | | | |
|---|---|---|---|---|---|---|
| | P2 | P3 | P4 | P5 | A1 | A2 |
| Dicydanil (99%) | 5 | 5 | 5 | 5 | 5 | 5 |
| Dodecylbenzenesulfonic acid | 5 | 6 | 7 | 9 | - | - |
| Dowanol DPM | 80 | 80 | 80 | 80 | 80 | 80 |
| Water | 10 | 9 | 8 | 6 | 15 | 11.9 |
| Lactic acid (80%) | - | - | - | - | - | 3.1 |
| The acid/dicyclanil molar ratio | 0.58 | 0.70 | 0.82 | 1.05 | 0.00 | 1.05 |

## Claims

1. 4,6-diamino-2-cyclopropylaminopyrimidine-5-carbonitrile dodecylbenzenesulfonate.

2. A method of preparing 4,6-diamino-2-cyclopropylaminopyrimidine-5-carbonitrile dodecylbenzenesulfonate, **characterized in that** 4,6-diamino-2-cyclopropylaminopyrimidine-5-carbonitrile is reacted with dodecylbenzenesulfonic acid.

3. The method according to claim 2, **characterized in that** said reaction is carried out in a glycol ether/water solvent system, wherein the preferred glycol ether is selected from the group comprising dipropylene glycol monomethyl ether, diethylene glycol monoethyl ether and mixtures thereof in all ratios.

4. The method according to claim 2 or 3, **characterized in that** said reaction is carried out *in situ* and the molar ratio of dodecylbenzenesulfonic acid to 4,6-diamino-2-cyclopropylaminopyrimidine-5-carbonitrile in said reaction is preferably from 0.50 to 1.10, and more preferably from 0.70 to 0.98.

5. An antiparasitic, preferably larvicide, composition comprising 4,6-diamino-2-cyclopropylaminopyrimidine-5-carbonitrile dodecylbenzenesulfonate.

6. The antiparasitic composition according to claim 5, **characterized in that** the content of 4,6-diamino-2-cyclopropylaminopyrimidine-5-carbonitrile dodecylbenzenesulfonate is from 1 to 25% by weight, preferably from 10 to 15% by weight, of the composition.

7. The antiparasitic composition according to claim 5 or 6, **characterized in that** it comprises castor oil in an amount of from 1 to 10% by weight of the composition.

8. The antiparasitic composition according to claim 5 or 6 or 7, **characterized in that** it further comprises a glycol ether selected from the group comprising dipropylene glycol monomethyl ether and diethylene glycol monoethyl ether and/or mixtures thereof in any ratio, in an amount of from 50 to 90% by weight of the composition and water in an amount of up to 10% by weight of the composition.

9. 4,6-diamino-2-cyclopropylaminopyrimidine-5-carbonitrile dodecylbenzenesulfonate for use in combating and controlling of parasites, in particular diptera larvae, in particular larvae of blow flies of the *Calliphoridae* family, in particular larvae of *Lucilia cuprina* and *Lucilia sericata,* in humans and animals.

10. 4,6-diamino-2-cyclopropylaminopyrimidine-5-carbonitrile dodecylbenzenesulfonate for use according to claim 9, wherein said 4,6-diamino-2-cyclopropylaminopyrimidine-5-carbonitrile dodecyl benzene sulfonate is applied topically to a human or animal body.

## Patentansprüche

1. 4,6-Diamino-2-Cyclopropylaminopyrimidin-5-Carbonitril-Dodecylbenzolsulfonat.

2. Ein Verfahren zur Herstellung von 4,6-Diamino-2-Cyclopropylaminopyrimidin-5-Carbonitril-Dodecylbenzolsulfonat, **dadurch gekennzeichnet, dass** 4,6-Diamino-2-Cyclopropylaminopyrimidin-5-Carbonitril mit Dodecylbenzolsulfonsäure umgesetzt wird.

3. Das Verfahren nach Anspruch 2, **dadurch gekennzeichnet, dass** die Reaktion in einem Glykolether/Wasser-Lösungsmittelsystem durchgeführt wird, wobei der bevorzugte Glykolether aus der Gruppe ausgewählt ist, die Dipropylenglykol-Monomethylether, Diethylenglykol-Monoethylether und Mischungen davon in verschiedenen Verhältnissen.

4. Das Verfahren nach Anspruch 2 oder 3, **dadurch gekennzeichnet, dass** die Reaktion in situ durchgeführt wird und das Molverhältnis von Dodecylbenzolsulfonsäure zu 4,6-Diamino-2-Cyclopropylaminopyrimidin-5-Carbonitril in der Reaktion vorzugsweise von 0,50 bis 1,10, und besonders bevorzugt von 0,70 bis 0,98 beträgt.

5. Die antiparasitäre, vorzugsweise larvizide Zusammensetzung, umfassend 4,6-Diamino-2-Cyclopropylaminopyrimidin-5-Carbonitril-Dodecylbenzolsulfonat.

6. Die antiparasitäre Zusammensetzung nach Anspruch 5, **dadurch gekennzeichnet, dass** der Gehalt an 4,6-Diamino-2-Cyclopropylaminopyrimidin-5-Carbonitril-Dodecylbenzolsulfonat 1 bis 25 Gew.-%, vorzugsweise 10 bis 15 Gew.-%, der Zusammensetzung beträgt.

7. Die antiparasitäre Zusammensetzung nach Anspruch 5 oder 6, **dadurch gekennzeichnet, dass** sie Rizinusöl in einer Menge von 1 bis 10 Gew.-% der Zusammensetzung enthält.

8. Die antiparasitäre Zusammensetzung nach Anspruch 5 oder 6 oder 7, **dadurch gekennzeichnet, dass** sie ferner einen Glykolether, ausgewählt aus der Gruppe, die Dipropylenglykol-Monomethylether und Diethylenglykol-Monoethylether und/oder Mischungen davon in verschiedenen Verhältnissen umfasst, in einer Menge von 50 bis 90 Gew.-% der Zusammensetzung und Wasser in einer Menge von bis zu 10 Gew.-% der Zusammensetzung umfasst.

9. 4,6-Diamino-2-Cyclopropylaminopyrimidin-5-Carbonitril-Dodecylbenzolsulfonat zur Verwendung bei der Bekämpfung und Kontrolle von Parasiten, insbesondere Diptera-Larven, insbesondere Larven von Schmeißfliegen der Familie Calliphoridae, insbesondere Larven von Lucilia cuprina und Lucilia sericata, bei Mensch und Tier.

10. 4,6-Diamino-2-Cyclopropylaminopyrimidin-5-Carbonitril-Dodecylbenzolsulfonat zur Verwendung nach Anspruch 9, wobei das 4,6-Diamino-2-Cyclopropylaminopyrimidin-5-Carbonitril-Dodecylbenzolsulfonat topisch auf einen menschlichen oder tierischen Körper aufgetragen wird.

## Revendications

1. 4,6-diamino-2-cyclopropylaminopyrimidine-5-carbonitrile dodécylbenzènesulfonate.

2. Une méthode de préparation du 4,6-diamino-2-cyclopropylaminopyrimidine-5-carbonitrile dodécylbenzènesulfonate, **caractérisée en ce que** le 4,6-diamino-2-cyclopropylaminopyrimidine-5-carbonitrile réagit avec l'acide dodécylbenzènesulfonique.

3. La méthode selon la revendication 2, **caractérisée en ce que** ladite réaction est effectuée dans un système de solvants éther de glycol/eau, dans lequel l'éther de glycol préféré est choisi dans le groupe comprenant l'éther monométhylique du dipropylène glycol, l'éther monoéthylique du diéthylène glycol et leurs mélanges dans tous les ratios.

4. La méthode selon la revendication 2 ou 3, **caractérisée par le fait que** ladite réaction est effectuée in situ et que le rapport molaire entre l'acide dodécylbenzènesulfonique et le 4,6- diamino-2-cyclopropylaminopyrimidine-5-carbonitrile dans ladite réaction est de préférence de 0,50 à 1,10, et plus préférentiellement de 0,70 à 0,98.

5. Une composition antiparasitaire, de préférence larvicide, comprenant le 4,6-diamino-2- cyclopropylaminopyrimidine-5-carbonitrile dodécylbenzènesulfonate.

6. La composition antiparasitaire selon la revendication 5, **caractérisée en ce que** la teneur en 4,6- diamino-2-cyclopropylaminopyrimidine-5-carbonitrile dodécylbenzènesulfonate est de 1 à 25 % en poids, de préférence de 10 à 15 % en poids, de la composition.

7. La composition antiparasitaire selon la revendication 5 ou 6, **caractérisée par le fait qu'**elle comprend de l'huile de ricin à raison de 1 à 10 % en poids de la composition.

8. La composition antiparasitaire selon les revendications 5 ou 6 ou 7, **caractérisée en ce qu'**elle comprend en outre un éther de glycol choisi dans le groupe comprenant l'éther monométhylique du dipropylène glycol et l'éther monométhylique du diéthylène glycol et/ou leurs mélanges dans n'importe quel ratio, en quantité de 50 à 90 % en poids de la composition et de l'eau en quantité allant jusqu'à 10 % en poids de la composition.

9. Le 4,6-diamino-2-cyclopropylaminopyrimidine-5-carbonitrile dodécylbenzènesulfonate à utiliser pour combattre et contrôler les parasites, en particulier les larves de diptères, en particulier les larves de mouches à viande de la famille des Calliphoridae, en particulier les larves de Lucilia cuprina et Lucilia sericata, chez l'homme et les animaux.

10. Le 4,6-diamino-2-cyclopropylaminopyrimidine-5-carbonitrile dodécylbenzènesulfonate à utiliser selon la revendication 9, dans lequel ledit 4,6-diamino-2-cyclopropylaminopyrimidine-5-carbonitrile dodécylbenzènesulfonate est appliqué par voie topique sur le corps d'un être humain ou d'un animal.
